# EUROPEAN PATENT APPLICATION

(11) **EP 1 741 438 A1**
(43) Date of publication of application: **10.01.2007**
(21) Application number: 05734379.0
(22) Date of filing: 20.04.2005
(51) Int. Cl.: A61K 35/74, A61K 9/107, A61K 9/19, A61K 39/04, A61K 39/39, A61K 47/02, A61K 47/22, A61K 47/26, A61K 47/44, A61P 35/00, A61P 37/04, A61P 43/00

(54) **PHARMACEUTICAL PREPARATION CONTAINING BACTERIAL CELL WALL SKELETON COMPONENT**

(30) Priority: 22.04.2004 JP 2004126827
(71) Applicant: Dainippon Sumitomo Pharma Co., Ltd., Osaka 541-8524 (JP)
(72) Inventor: NOMURA, T., Dainippon Sumitomo Pharma Co., Ibaraki-shi, Osaka 567-0878 (JP)
(74) Representative: Vossius & Partner
(86) International application number: PCT/JP2005/007523
(87) International publication number: WO 2005/102369

(57) **Abstract**

The present invention provides a lyophilized formulation comprising bacterial cell wall skeleton, an oil and a surfactant, **characterized in that** it comprises an antioxidant; for example, an oil-in-water emulsion formulation comprising BCG-CWS and an oil and a lyophilized formulation thereof which contains tocopherols as an antioxidant and further a buffer agent consisting of phosphate, whereby the pH of an oil-in-water emulsion obtainable by rehydrating said lyophilized formulation in water is adjusted to pH 5.5 - 8.5.

## Description

### TECHNICAL FIELD

The present invention relates to a highly stable pharmaceutical composition, in particular a lyophilized formulation which comprises bacterial cell wall skeleton (hereinafter, "bacterial cell wall skeleton" may be abbreviated as "bacteria-CWS, and cell wall skeleton as "CWS").

### BACKGROUND ART

It has been acknowledged that bacterial cell wall skeleton (bacteria-CWS) have an immunostimulatory activity and show antitumor effects in a experimental tumor system using animal models and also in cancer immunotherapy for human. It has also been acknowledged that, when the above-mentioned bacterial cell wall skeleton are dispersed in oil, emulsified and administered in an oil-in-water emulsion formulation, the anti-tumor effect or the like due to the immuostimulatory activity greatly increases.

For example, it has been reported that cancer immunotherapy using an oil-in-water emulsion of bacterial cell wall skeleton of *Bacille Calmette-Guerin* (BCG), which may hereinafter be referred to as "BCG-CWS", achieved excellent results in the treatment of human cancer (see, Non-patent documents 1 and 2).

Such an oil-in-water emulsion formulation can be prepared by adding water containing a surfactant to a paste obtained by dispersing bacteria-CWS in an oil (see, Non-patent documents 3, 4 and 5). However, an oil-in-water emulsion formulation comprising bacteria-CWS is generally unstable and, therefore, it is necessary to prepare manually a small amount of oil-in-water emulsion formulation just before use in the clinical practice. In fact, it is difficult to prepare constantly formulations with uniform quality by manual operations. Further, such formulations adapted to be prepared before use can hardly be commercialized as pharmaceutical preparations.

Under the conditions, methods for preparing an oil-in-water emulsion formulation by reconstituting a lyophilized formulation, which had been prepared by lyophilizing an oil-in-water emulsion formulation, in a dispersing solvent just before use have been examined. Such lyophilized formulations have been reported (see, Patent documents 1 and 2).

The following matters, for example, are significant for providing a lyophilized formulation comprising bacteria-CWS as a pharmaceutical preparation: (1) the lyophilized formulation has the long-term storage stability requisite for pharmaceutical preparations, and (2) an emulsion obtained by adding a dispersing solvent such as injectable distilled water to the lyophilized formulation is substantially equivalent to the oil-in-water emulsion before lyophilization. However, known lyophilized formulations have drawbacks, for example, a decreasing tendency of bacteria-CWS content under harsh conditions such as high temperature.

Therefore, lyophilized formulations of bacteria-CWS having an improved storage stability have been demanded.

Antioxidants such as tocopherol have been widely used in liquid preparations including medicines and cosmetics which contain a substance prone to oxidization for the purpose of stabilization; however, nothing has been known about inhibitory effects on the degradation of bacteria-CWS.
Patent document 1: WO00/3724
Patent document 2: WO2004/01271
Non-patent document 1: A. Hayashi et al., Pro. Japan Acad., 70, Ser. B 205-209 (1994)
Non-patent document 2: A. Hayashi et al., Pro. Japan Acad., 74, Ser. B 20550-55 (1998)
Non-patent document3: J. Nat. Cancerlnst. 48, 831-835 (1972)
Non-patent document 4: J. Bacteriol, 92, 869-879 (1966)
Non-patent document 5: Gann, 69, 619-626 (1978)

### DISCLOSURE OF THE INVENTION

The purpose of the present invention is to provide a pharmaceutical composition comprising bacterial cell wall skeleton as an active ingredient, in particular, a lyophilized formulation, which shows an excellent storage stability.

The present inventors have conducted an intensive study for developing lyophilized formulations comprising bacteria-CWS and oil into medicinal products.

It has been known that such lyophilized formulations can be prepared according to the method described in the above-mentioned Patent documents 1 and 2. The process comprises: (1) preparing an oil mixture of bacteria-CWS and an oil such as squalane using an organic solvent, (2) adding one of polysorbates as a surfactant, (3) adding mannitol as an excipient, (4) emulsifying the mixture of (3) in water to give oil-in-water emulsion of high uniformity; and lyophilizing the resultant oil-in-water emulsion to yield a lyophilized formulation.

However, when such a lyophilized formulation was examined for the long-term storage stability requisite for pharmaceutical preparations, it was found that bacteria-CWS structure was partially degradated and mycolic acid which is one of constituents was released. Besides, when water is added to such a lyophilized formulation after long-term storage for reconstitution (rehydration), the resulting oil-in-water emulsion showed pH shift toward acidic site. From these findings, it was revealed that improvement of storage stability is inevitable to develop a pharmaceutical composition comprising as an active ingredient bacteria-CWS into a medicinal product.

The present inventors have conducted an intensive study and found that the stability of a lyophilized formulation comprising bacteria-CWS can be extremely improved by adding an antioxidant. The present inventors have also found that the stability of a lyophilized formulation can be synergistically improved by adding a buffer agent during preparation of oil-in-water emulsion as an intermediate and adjusting the emulsion solution at pH 5.5 - 8.5. Thus, the present inventors have found that the long-term storage stability of a lyophilized formulation can be dramatically improved by adding an antioxidant, and preferably further adding a buffer agent.

The present invention has been established on the basis of the above-mentioned findings.

Thus, the present invention is related to:
[1] A lyophilized formulation of an oil-in-water emulsion comprising bacterial cell wall skeleton, an oil and a surfactant, characterized in that it further comprises an antioxidant;
[2] The lyophilized formulation according to [1], wherein the surfactant is polyoxyethylene sorbitan fatty acid ester;
[3] The lyophilized formulation according to [1] or [2], wherein the antioxidant is tocopherol;
[4] The lyophilized formulation according to any one of [1] to [3], characterized in that it further contains a buffer agent so that the pH of an oil-in-water emulsion comprising bacterial cell wall skeleton at a concentration of 0.5 - 1 mg/ml obtainable by rehydrating said lyophilized formulation in water is adjusted to pH 5.5-8.5;
[5] The lyophilized formulation according to [4], wherein the buffer agent is a phosphate;
[6] The lyophilized formulation according to [5], characterized in that it is adjusted so that the concentration of the buffer agent in an oil-in-water emulsion comprising bacterial cell wall skeleton at a concentration of 0.5 - 1 mg/ ml obtainable by rehydrating said lyophilized formulation in water is about 10 - 20 mM;
[7] The lyophilized formulation according to any one of [1] to [6], characterized in that it further comprises mannitol as an excipient.
[8] The lyophilized formulation according to any one of [1] to [7], wherein the bacterial cell wall skeleton are *Mycobacterium bovis Bacille Calmette-Guerin* cell wall skeleton.
[9] The lyophilized formulation according to any one of [1] to [8], which has characteristics (1) and (2) below:
   (1) the amount of free mycolic acid after 30-day storage at 80°C is about 2 % or less, and
   (2) the pH of an oil-in-water emulsion comprising bacterial cell wall skeleton at a concentration of 0.5 - 1 mg/ml obtainable by rehydrating said lyophilized formulation in water after 30-day storage at 80°C is substantially equal to the pH before storage.
[10] An oil-in-water emulsion obtainable by adding an aqueous solvent to a lyophilized formulation described in any one of [1] to [9];
[11] An oil-in-water emulsion comprising bacterial cell wall skeleton, an oil and a surfactant, characterized in that it further comprises an antioxidant;
[12] The oil-in-water emulsion according to [11], wherein the surfactant is polyoxyethylene sorbitan fatty acid ester;
[13] The oil-in-water emulsion according to [11] or [12], wherein the antioxidant is tocopherol;
[14] The oil-in-water emulsion according to any one of [11] to [13], characterized in that it further contains a buffer agent and is adjusted to pH 5.5 - 8.5;
[15] The oil-in-water emulsion according to [14], wherein the buffer agent is a phosphate;
[16] The oil-in-water emulsion according to [14] or [15], characterized in that it is adjusted so that the concentration of the buffer agent is about 10 - 20 mM;
[17] The oil-in-water emulsion according to any one of [11] to [16], characterized in that it further comprises mannitol as an excipient.
[18] The oil-in-water emulsion according to any one of [11] to [17], wherein the bacterial cell wall skeleton are *Mycobacterium bovis Bacille Calmette-Guerin* cell wall skeleton.
[19] A lyophilized formulation obtainable by lyophilizing an oil-in-water emulsion described in any one of [11] to [18].
[20] A method of stabilizing the above-mentioned lyophilized formulation comprising bacterial cell wall skeleton, which comprises adding a stabilizing agent containing an antioxidant to the lyophilized formulation and thereby conferring a long-term storage stability on the above-mentioned bacterial cell wall skeleton in the lyophilized formulation;
[21] The method according to [20], which further comprises adding a buffer agent so as to adjust the pH of an oil-in-water emulsion comprising bacterial cell wall skeleton at a concentration of 0.5 - 1 mg/ml obtainable by rehydrating said lyophilized formulation to pH 5.5 - 8.5;
[22] A method of inhibiting degradation of a bacterial cell wall skeleton in a lyophilized formulation comprising bacterial cell wall skeleton, an oil and a surfactant, characterized in that an antioxidant is also added to the lyophilized formulation;
[23] The method according to any one of [20] to [22], wherein the bacterial cell wall skeleton is BCG-CWS, the oil is squalane, the surfactant is Polysorbate 80, and the antioxidant is tocopherol;
[24] A stabilization accelerator for an oil-in-water emulsion comprising bacterial cell wall skeleton, squalane and a surfactant or a lyophilized formulation of the emulsion, characterized in that it comprises tocopherol as an active ingredient;
[25] The stabilization accelerator according to [24], characterized in that it further comprises a buffer agent so that the pH of an oil-in-water emulsion before lyophilization which contains bacterial cell wall skeleton at a concentration of 0.5 - 1 mg/ml or the pH of an oil-in-water emulsion which is obtainable by rehydrating the lyophilized formulation and contains bacterial cell wall skeleton at a concentration of 0.5 - 1 mg/ml is adjusted to pH 5.5 - 8.5;
[26] The stabilization accelerator according to [25], characterized in that the buffer agent is a phosphate.

The present invention makes it possible to provide a lyophilized formulation comprising as an active ingredient bacteria CWS and having an excellent long term storage stability. The said lyophilized formulation is useful as a therapeutic agent in the cancer immunotherapy.

### BEST MODE FOR CARRYING OUT THE INVENTION

As used herein, the term "bacterial cell wall skeleton" (it may be herein abbreviated as bacteria-CWS") refers to any microorganism-derived bacterial components comprising cell wall skeleton (Cell Wall Skeleton; CWS), including bacterial body itself of microorganisms such as dead bacterium of *Mycobacterium tuberculosis* as well as cell wall skeleton isolated (purified) to a certain extent. Examples of microorganisms from which CWS is derived include gram-positive rods such as bacteria of *Mycobacteria, Nocardia, Corynebacteria, Rhodococcus, Gordona* and the like. *"Mycobacteria"* represents bacteria belonging to acid-fast bacteria, *Mycobacterium,* and particularly includes *Mycobacterium tuberclosis, Mycobacterium bovis* (including BCG, *Bacillus Calmette-Guerin), Mycobacterium africanum*, *Mycobacterium microti,* as well as *Mycobacterium leprae,* non-tuberclosis acid-fast bacteria such as *Mycobacterium* kansasii, *Mycobacterium avium, Mycobacterium phlei* and the like. Above all, BCG, one of *Mycobacterium bovis,* and *Nocardia rubra,* one of *Nocarida,* are preferred.

The "cell wall skeleton, CWS" refers to insoluble residue obtainable by a purification process which comprises physically crashing bacterial cells, removing nucleic acids with nuclease, deproteinizing with protease, and washing with an organic solvent for defatting The method for the preparation is well known in the art (J. Natl. Cancer Inst., 52, 95-101 (1974)).

In the present invention, the concentration of bacteria-CWS in water-in-oil emulsion is 0.01 - 10 mg/ml. Preferably, the concentration is from 0.1 mg/ml to 2 mg/ml, more preferably from 0.2 mg/ml to 1 mg/ml, and still more preferably from 0.5 mg/ ml to 1 mg/ml.

In the present invention, "oil" includes mineral oil, an animal oil and an vegetable oil, as described in Immunology, 27, 311-329 (1974). Mineral oil is exemplified by a liquid paraffin (Drakeol-6VR, Moresco-violess U-6, Molesco-violess U-8, etc.), bayol (Bayol F), and the like. Vegetable oil is exemplified by soybean oil, synthelane 4, ethyl oleate, peanut oil, camellia oil, sesame oil, AD-65 (a mixture of peanut oil, Arlacel, and aluminum monostearate), and the like. Animal oil is exemplified by terpenoid derivatives such as squalane, squalene, and the like. A mixture of oils selected from animal oils, vegetable oils, mineral oils may be included. Among them, squalane; a mixture of squalane with a vegetable oil(s) selected from, for example, soybean oil, ethyl oleate and oleic acid or oils derived therefrom; Drakeol-6VR; and a mixture of squalane with a mineral oil(s) selected from, for example, various liquid paraffin, are preferred.

More preferred examples include squalane, Drakeol-6VR, a mixture of squalane and soybean oil, a mixture of squalane and ethyl oleate, or a mixture of squalane and Drakeol-6VR. Squalane is still more preferred.

The concentration of oil is adjusted appropriately so that the viscosity of the hereinafter stated paste (oily mixture) of bacteria-CWS and oil is about 0.7 poise or less (25 °C), preferably about 0.2 - 0.6 poise (25 °C) and more preferably about 0.28 - 0.55 poise (25°C). When a mixture of more than one oils is used, respective oils may be mixed at an appropriate composition ratio. In that case, the composition ratio is determined so that the viscosity is kept at 0.2 - 0.7 poise (25 °C) during the process of mixing the oil mixture with bacteria-CWS.

For example, when squalane is used as an oil, a paste comprising bacteria-CWS preferably has a viscosity from about 0.35 to 0.55 poise (25 °C), and more preferably, from about 0.39 to 0.51 poise (25 °C). Particular examples include compositions containing about 6.6 - 35.2 g, preferably about 8.4 - 35.2 g of squalane per about 0.66 g of bacteria-CWS.

The above-mentioned paste may be prepared by the following steps 1) and 2):
1) a step of mixing bacteria-CWS and oil with stirring in an organic solvent as a dispersion-aiding solvent agent; and
2) a step of evaporating the organic solvent in step 1).

Examples of organic solvent include hydrocarbon solvents such as heptane and toluene, hydrocarbon solvents (e.g., heptane and the like) containing 5 to 20 % of alcohol solvent (e.g., ethanol and the like), and halogenated hydrocarbon solvents such as 1,2-dichloroethane, chloroform and the like. The method for preparing a paste, and organic solvents to be used in the step 1) are shown in WO2004/012751.

A "surfactant" which can be used in the present invention is not limited to a particular species as long as it is used in a pharmaceutical formulation. Examples include phospholipids, nonionic surfactants, and the like. The phospholipid includes phosphatidyl amine, phosphatidyl ethanol amine, phosphatidyl inositol, phosphatidyl serine, sphingomyelin, lecithin, or the like. A hydrogenated phospholipid is also usable. The nonionic surfactant includes polyoxyethylene-polyoxypropylene copolymers, polyoxyethylene hydrogenated castor oil derivatives, polyoxyethylene castor oil derivatives, polyoxyethylene sorbitan fatty acid esters and sorbitan fatty acid esters. The polyoxyethylene sorbitan fatty acid ester includes polyoxyethylene sorbitan monolaurate (Polysorbate 20), polyoxyethylene sorbitan monopalmitate (Polysorbate 40), polyoxyethylene sorbitan monostearate (Polysorbate 60), polyoxyethylene sorbitan monooleate (Polysorbate 80), and the like. The sorbitan fatty acid ester includes sorbitan monolaurate (Span 20), sorbitan monopalmitate (Span 40), sorbitan monostearate (Span 60), sorbitan monooleate (Span 80), and the like.

Preferred surfactants include egg yolk phosphatidylcholine, egg yolk lecithin, soybean lecithin, Polysorbate 80, Polysorbate 20, polyoxyethylene hydrogenated castor oil 60 (HCO-60), polyoxyethylene hydrogenated castor oil 50 (HCO-50), polyoxyethylene (160)polyoxypropylene(30)glycol (Pluronic F68) and the like. More preferred surfactant includes polyoxyethylene sorbitan fatty acid esters such as Polysorbate 80, Polysorbate 40, Polysorbate 60 and the like. Polysorbate 80 is especially preferred.

The concentration of surfactant in oil-in-water emulsion is suitably in the range of 0.01 - 10 %w/w, preferably 0.01 - 3 %w/w, and more preferably 0.05 - 1 %w/w. The surfactant may be used as a single species or as a combination of several species, as appropriate.

The "antioxidant" in the present invention refers to a compound having a radical-inhibitory activity of inhibiting the chain reaction (autooxidation) or a peroxide-decomposing activity which means inactivating peroxides through non-radical decomposition. Any antioxidants can be used without limitation as long as they can be used in pharmaceutical preparations. Concrete examples include tocopherols, butylhydroxytoluene (hereinafter, referred to as "BHT"), butylhydroxyanisole (hereinafter, referred to as "BHA"), nordihydroguaiaretine, propyl gallate, a fatty acid ester of Vitamin C, sorbic acid, and the like. Preferred antioxidants are tocopherols.

Examples of tocopherols include α-tocopherol, β-tocopherol, γ-tocopherol, δ-tocopherol, or a mixture of 2 to 4 species of tocopherols selected arbitrarily from them. Concrete examples include α-tocopherol (trade name: E-mix 80, E-mix 70L, etc.).

The amount of antioxidant to be formulated in the present invention is preferably 0.0001 %w/w or greater, more preferably 0.001 %w/w or greater of the whole composition in order to prevent oxidative degradation of raw materials sufficiently. Although there are no limitations regarding the upper limit of the amount, it can generally be 10.0 %w/w or less of the whole composition. The concentration range of antioxidant in an oil-in-water emulsion is 0.00001 - 10 %w/w, preferably 0.0001 - 5 %w/w. Such an antioxidant may be used as a single species or as a combination of several species, as appropriate

An antioxidant may be added at any step during the process for the preparation of oil-in-water emulsion. However, it is preferably added when preparing an oily mixture (paste) of bacteria-CWS and oil by previously dissolving in the oil

The "excipient" is a component used for the purpose of maintaining and improving the stability of the above-mentioned emulsion as an emulsion or a lyophilized formulation. Examples of excipients usable in the present invention include monosaccharides, sugar alcohols, polysaccharides, amino acids, proteins, urea, or inorganic salts. The monosaccharide and disaccharide include glucose, fructose, sucrose, lactose, trehalose and the like. The sugar alcohol includes mannitol, sorbitol and the like. Preferred polysaccharides include dextran, starch, maltodextrin, cellulose, polyvinylpyrrolidone, sodium alginate, or the like. Preferred amino acids include neutral amino acids such as alanine, glycine, proline and the like, and more preferred neutral amino acids include glycine and the like. Preferred proteins include albumin, gelatin, collagen and the like. Inorganic salts include sodium chloride, potassium chloride, calcium chloride, sodium sulfate, sodium carbonate and the like.

Preferred excipients include monosaccharides or sugar alcohols, and more preferred excipient is mannitol.

Such excipients may be used as a single species or as a combination of several species, as appropriate.

The concentration of excipient in an oil-in-water emulsion is suitably in the range of 0.1 - 20 %w/w, and preferably 0.1 - 10 %w/w. The suitable concentration of excipient varies depending on the types of excipient, and may be adequately adjusted in view of the manufacturing scale or the contents of respective components. The suitable concentration for amino acids such as glycine is in the range of 2.25 % (300 mM) to 11.25 % (1500 mM), and preferably about 6.75 % (900 mM). In the case of mannitol, the concentration thereof in an oil-in-water emulsion can be preferably in the range of 1 - 10 %, more preferably 1-8 %, and even more preferably about 3 - 6 %. Since mannitol can be used as an excipient at an almost equal concentration to that makes the product isotonic, it is possible to prepare a stable formulation with less burden on a living body by use of mannitol.

Those described as excipients in the above may be used as an isotonic agent, if needed. The excipient or isotonic agent can be used as a single species or as an appropriate combination of several species. Although the excipient above generally serves as an isotonic agent, any other agent can be added as an isotonic agent. The concentration of the isotonic agent may be determined depending on the contents of other components; however, suitable concentration is generally 0.1 - 30 %w/w, preferably 1- 10 %w/w.

Examples of "buffer agent" as described herein include, specifically, amino acids such as glycine, alanine, arginine, glutamine, cysteine, serine, threonine, valine, histidine, phenylalanine, methionine, aspartic acid, glutamic acid, ε-aminocaproic acid, lysine, leucine, and the like, or salts thereof such as hydrochlorides, sodium salts, potassium salts, and the like; ampholytes such as nicotinamide, aminobenzoic acid, and the like, or salts thereof such as sodium salts; salts such as sodium and potassium salts of a weak acid including citric acid, phosphoric acid, lactic acid, acetic acid, boric acid, propionic acid, butyric acid, valeric acid, glycolic acid, oxalic acid, malonic acid, succinic acid, glutaric acid, adipic acid, malic acid, fumaric acid, maleic acid, benzoic acid, salicylic acid, phthalic acid, tartaric acid, and the like; combinations of a salt of weak acid above and a weak acid or a strong acid such as hydrochloric acid, sulfuric acid, and the like; Tris-buffer solution, and the like.

Examples of phosphate buffer solution includes a buffer solution consisting of 5 - 50 mM sodium dihydrogenphosphate and disodium hydrogenphosphate. In general, sodium dihydrogenphosphate and disodium hydrogenphosphate are dissolved at the ratio of 1 : 2.3 (w/w), and the pH may be finely adjusted with an acid such as hydrochloric acid or a base such as sodium hydroxide, if necessary. Examples of Tris buffer solution include a buffer solution consisting of 5 - 50 mM Tris solution, and the pH may be finely adjusted with 0.1 - 4 N hydrochloric acid, or the like, if necessary. Examples of citrate buffer solution includes a buffer solution consisting of 5 mM - 50 mM citrate solution, and the pH may be finely adjusted with 0.1 - 4 N hydrochloric acid, or the like, if necessary. A phosphate buffer solution is preferred.

The amount of a buffer agent to be formulated in the present invention is not limited particularly on condition that it is sufficient for the buffer agent to exert adequate buffer capacity. Preferably, it can be 5 - 20 mM, more preferably 10 - 20 mM.

The amount of a buffer agent may be adjusted properly when a substance with buffering effect such as amino acid is used.

The lyophilized formulation of the present invention shows an excellent long-term storage stability, and has the following characteristics:
(1) The amount of free (released) mycolic acid after 30-day storage at 80 °C is about 2 % or less.
(2) The pH of an oil-in-water emulsion containing bacterial cell wall skeleton at a concentration from 0.5 mg/ml to 1 mg/ml obtained by rehydration of said lyophilized formulation in water after 30-day storage at 80 °C is substantially equal to the pH measured before storage.

More preferably, the amount of free mycolic acid set forth in (1) is within about 1 %. Further, the term "substantially equal" set forth in (2) means that the difference of pH values of oil-in-water emulsions obtained by reconstituting a lyophilized formulation in water before and after storage, respectively, is within 0.8 preferably within 0.5.

The lyophilized formulation of the present invention can provide an oil-in-water emulsion formulation by reconstituting (rehydrating) in an aqueous solvent. The said oil-in-water emulsion is also encompassed within the scope of the present invention.

The aqueous solvent to be used for reconstituting the lyophilized formulation of the present invention serves as a dispersion solvent for emulsion particles and includes distilled water for injection, physiological saline, a buffer solution or the like, but is not limited thereto on condition that it is an injectable aqueous solvent. When aqueous solvents used for reconstitution of lyophilized formulation are those containing an inorganic salt, such as physiological saline, buffer solution or the like, the composition of said aqueous formulation can be selected appropriately considering the stability or the like of the oil-in-water emulsion after reconstitution. The concentration of an inorganic salt which is added as a component of a buffer agent or an excipient in the oil-in-water emulsion after reconstitution is preferably adjusted so that the formulation becomes isotonic at the time of administration to human.

The present invention also encompasses an oil-in-water emulsion comprising bacteria-CWS, an oil, a surfactant and an antioxidant, which is an intermediate in the preparation of lyophilized formulations of the present invention.

The said "oil-in-water emulsion" may further contain an excipient, a buffer agent, and the like. Specifically, the oil-in-water emulsion preferably contains an excipient such as mannitol or the like, and is adjust the pH to 5.5 - 8.5 by adding a buffer agent.

The oil-in-water emulsion is characterized in that it can be prepared according to the method described in the above-described WO 04/012751, which comprises the following steps:
1) a step of mixing bacteria-CWS and an oil with stirring in an organic solvent as a dispersion-aiding solvent;
2) a step of evaporating the organic solvent used in 1) to prepare a paste containing bacteria-CWS; and
3) a step of emulsifying the resulting paste (oily mixture) comprising bacteria-CWS and oil together with a surfactant in water. As mentioned above, an antioxidant can be added in step 1) or 3), however, it is preferably added in step 1). Further, an excipient is preferably added in step 3).

Examples of organic solvent to be used in the above include toluene, heptane, a mixed solvent of heptane-ethanol and the like. The method of preparing bacteria-CWS-containing paste is described in WO 00/3724 and WO 2004/012751.

The oil-in-water emulsion as a manufacturing intermediate or the oil-in-water emulsion formulation obtained by reconstituting the lyophilized formulation of the present invention is characterized in that it comprises the above stated bacteria-CWS-containing paste, 0.1 - 20 % preferably 1 - 10 % of excipient, 0.0 1 - 10 % preferably 0.01 - 3 % of surfactant, and 0.001 - 10 % preferably 0.001 - 0.1 % of antioxidant.

Specifically, the oil-in-water emulsion is characterized in that it contains 0.67 - 3.35 g/2L of bacteria-CWS, 0.1 - 10 %w/w, preferably 0.4 - 8 %w/w, more preferably 0.6 - 5 %% of squalane, 1 - 10 %w/w of stabilizer, 0.01 - 3 %w/w of surfactant, and 0.001 - 0.1 %w/w of antioxidant.

Examples of the above-mentioned aqueous solvent include water, physiological saline, a buffer solution, and the like.

The "buffer solution" above refers to an aqueous solution containing a buffer agent which is used for the purpose of maintaining pH of an oil-in-water emulsion formulation (inclusive of an emulsion obtained by reconstituting a lyophilized formulation in water). Any buffer solutions can be used in the present invention without limitation, but those used in the pharmaceutical preparation are preferred.

The oil-in-water formulation of the present invention is preferably adjusted to pH 5.5 - 8.5, preferably pH 6.0 - 7.0 with a buffer agent.

A lyophilized formulation can be prepared by lyophilizing the above-mentioned oil-in-water emulsion. Thus, the lyophilized formulation of the present invention can be prepared by lyophilizing oil-in-water emulsion, usually followed by nitrogen substitution of a vial and putting a stopper on the vial finally . There are no limitations regarding temperature, time and the like for lyophilization of oil-in-water emulsion, and methods described in, for example, WO 2000/3724 or WO04/012751 can be used.

The oil-in-water emulsion formulation of the present invention can be administered parenterally, for example, as injection. Dosage form varies depending on the purpose of treatment and is not limited to a particular form. Usually, it can be formulated as an injection for intradermal administration. The amount of bacteria-CWS in the emulsion is generally in the concentration range of 0.1 - 1.0 mg/ml, preferably 0.5 - 1.0 mg/ml. The lyophilized formulation of the present invention can be used after reconstituting in an appropriate amount of aqueous solvent such as, for example, water, physiological saline or a buffer solution to give the oil-in-water emulsion of the present invention. In case where an oil-in-water emulsion obtained by reconstituting a lyophilized formulation is administered to a living body, the concentrations of respective components in the emulsion may be different from the concentrations in the oil-in-water emulsion as a manufacturing intermediate before lyophilization. An appropriate volume of aqueous solvent may be preferably 0.5 to 4 times as much as the volume of emulsion before lyophilization.

Although the dosage and frequency of administration may vary depending on the disease to be treated, and the symptom, age, body weight, sex, and the like of a patient, the dosage for an adult is, for example, within the range of 10 - 250 µg, preferably 25 - 200 µg per administration, which can be administered once in every week or in every four weeks.

The lyophilized formulation and the oil-in-water emulsion formulation obtainable by rehydrating the said lyophilized formulation of the present invention are useful as a therapeutic or preventive agent for cancer, particularly, as an immunotherapeutic agent. Examples of cancer to be treated include, but not limited thereto, lung cancer, gastric cancer, hepatic cancer, pancreatic cancer, colon caner, uterine cancer, breast cancer, acute myelogenous leukemia, lingual cancer, pharyngeal cancer, ovarian cancer, brain cancer, and the like. In this context, the therapeutic agent for cancer includes a cancer metastasis inhibitor.

Further, the lyophilized formulation and the oil-in-water emulsion formulation obtainable by reconstituting the said lyophilized formulation of the present invention can be used in combination with other immunotherapeutic agents, specifically, cancer vaccine comprising as an active ingredient a various kinds of cancer antigens.

The present invention is further illustrated by the following examples, but should not be construed at being restricted by the same.

### Example 1

A solution of 1 mg/mL tocopherol (E mix 80 or E mix 70L) / 10 % ethanol/90 % heptane was prepared, and a portion (60 mL) thereof was mixed thoroughly with squalane (105.6 g) to give "mixture-A".

As bacterial cell wall skeleton, BCG-CWS (2770 mg) was added to a mixed solution of mixture-A (70.4 g) and 10% ethanol/90% heptane (400 mL), and allowed to disperse by shaking or ultrasonication at room temperature. The dispersed mixture was then heated at 70 °C under nitrogen flow or atmospheric air to evaporate-ethanol/heptane. After adding 0.02 %w/w Polysorbate 80/10 mM phosphate buffer solution (888.6 g), the mixture was roughly emulsified using a homomixer, which was followed by addition of aqueous 10 %w/w Polysorbate 80 solution (36.7 g) and then complete emulsification. The final concentration of Polysorbate 80 was adjusted to 0.1 %w/w by adding a solution of 10 w/w% Polysorbate 80 (1.5 g) to give an oil-in-water emulsion. To the emulsion was added an aqueous 6.7 w/w% mannitol/ 1.2 w/w% Polysorbate 80 solution (3000 g) to obtain the final product (4000 g).

The resulting oil-in-water emulsion formulation was dispensed into 2 mL aliquots in vials, and lyophilized to provide the lyophilized formulation of the present invention. Lyophilization was carried out using a lyophilizer (DFM-13A-S,ULVAC).

### Example 2

A solution of 1 mg/mL tocopherol (E mix 80 or E mix 70 L)/ 10 % ethanol/90 % heptane was prepared, and a portion (40 mL) thereof is mixed thoroughly with squalane (70.4 g) to give "mixture-B".

As bacterial cell wall skeleton, BCG-CWS (1358 mg) was added to a mixed solution of mixture-B (35.2 g) and 10% ethanol/ 90% heptane (200 mL), and allowed to disperse by shaking or ultrasonication at room temperature. The dispersed mixture was then heated at 70 °C under nitrogen flow or atmospheric air to evaporate ethanol/heptane. After adding 0.02 %w/w Polysorbate 80/10 mM phosphate buffer solution (444.3 g), the mixture was roughly emulsified using a homomixer, which was followed by addition of aqueous 10 %w/w Polysorbate 80 solution (18.4 g) and then complete emulsification. The final concentration of Polysorbate 80 was adjusted to 0.1 %w/w by adding a solution of 10 w/w% Polysorbate 80 (0.76 g) to give an oil-in-water emulsion. To the emulsion was added an aqueous 6.7 w/w% mannitol/ 1.2 w/w% Polysorbate 80 solution (1500 g) to obtain the final product (2000 g).

The resulting oil-in-water emulsion formulation was dispensed into 0.5 or 1 mL aliquots in vials, and lyophilized to provide the lyophilized formulation of the present invention. Lyophilization was carried out using a lyophilizer (DFM-05A-S, ULVAC).

### Example 3

### (Stability test)

Storage stability of lyophilized formulations comprising either an antioxidant or a buffer agent as prepared in Examples 1, 2, 5 and 6 and a lyophilized formulation (Reference Example 1) prepared according to the method of WO2004/012751 (Example 14) was evaluated. That is, each lyophilized formulation was put in an assembled box and stored in a gas-phase incubator kept at 80 °C. After one week, the formulation was taken out, and free (released) mycolic acid and pH after reconstituting in injectable distilled water were measured. The results are shown in Table 1.

**Table 1**

| Manufacturing Toco Scale | 10 ppm pherol Tocopherol | 10 mM Phosphate buffer solution | Before test | | 80°C, one week | |
|---|---|---|---|---|---|---|
| | | | pH | Free mycolic acid (%) | pH | Free mycolic acid (%) |
| 4000g(Ex.1) | ○ | ○ | 6.9 | 0.1 | 6.8* | 0.2* |
| 2000g (Ex. 2) | ○ | ○ | 6.9 | 0.3 | 6.8 | 0.3 |
| 240g (Ex. 5) | ○ | × | 6.3 | 0.1 | 6.7 | 1.0 |
| 240g (Ex. 6) | × | ○ | 6.9 | 0.3 | 6.5 | 1.5 |
| Ref. Ex. 1 | × | × | 6.5 | 0.2 | 3.5 | 19.7 |

| | | | | | | |
|---|---|---|---|---|---|---|
| *: Data of 1 month | | | | | | |

As can be seen from Table 1, in the formulation of Reference Example 1, the amount of free mycolic acid significantly increased and pH decreased greatly after one-week storage at 80 °C. On the contrary, in the lyophilized formulations of Examples 5 and 6, both of the amount of free mycolic acid and the decrease in pH were prevented. Further, in the lyophilized formulations of Examples 1 and 2 of the present invention which contains both of an antioxidant and a buffer agent, change in both of the amount of free mycolic acid and pH was hardly recognized, indicating that the long-term storage stability was greatly improved. In this context, the amount of free mycolic acid is expressed as a percentage of said acid in the total amount of BCG-CWS as measured according to the method described in WO2004/012751.

### Example 4

Lyophilized formulations comprising 5 mM, 10 mM, 20 mM, and 100 mM of phosphate buffer solution, respectively, were prepared in a similar manner to Example 1 except that ethanol-heptane mixed solvent without tocopherol was used. The storage stability was tested in a similar manner to Example 3. The results of measurement of pH are shown in Table 2.

**Table 2**

| Formula | pH | | | Free mycolic acid (%) | |
|---|---|---|---|---|---|
| | Before test | 80°C, one week | 80°C, one month | Before test | 80°C, one week |
| Distilled water for injection | 6.5 | 3.5 | 3.7 | 0.2 | 19.7 |
| 5mM Phosphate buffer solution | 6.7 | 5.3 | 6.0 | 0.1 | 2.0 |
| 10mM Phosphate | 6.8 | 6.0 | 6.5 | <0.1 | 1.8 |
| buffer solution | | | | | |
| 20mM Phosphate buffer solution | 6.7 | 6.1 | 6.6 | <0.1 | 1.8 |
| 100mM Phosphate buffer solution | 6.8 | 6.3 | - | <0.1 | 2.1 |

Further, properties of lyophilized cakes in oil-in-water emulsion formulations obtained by reconstituting lyophilized formulations in water after 1-week storage at 80°C are shown in Table 3 below.

**Table 3**

| Formula | Good | Shrink | Float | Total | Water content in formulation (%) |
|---|---|---|---|---|---|
| Distilled water for injection | 30 | 0 | 0 | 30 | 0.3 |
| 5 mM Phosphate buffer solution | 30 | 0 | 0 | 30 | 0.2 |
| 10 mM Phosphate buffer solution | 30 | 0 | 0 | 30 | 0.2 |
| 20 mM Phosphate buffer solution | 30 | 0 | 0 | 30 | 0.3 |
| 100 mM Phosphate buffer solution | 0 | 27 | 3 | 30 | 0.6 |

The figure in Tables shows the number of stored vials. Among 30 vials of respective groups, the number of vials showing change was counted.

The results in Table 2 show that a lyophilized formulation with excellent storage stability can be obtained by use of a phosphate buffer solution of 10 mM or higher concentration, in which the pH change as well as the amount of free mycolic acid are suppressed.

In addition, as can be seen from Table 3, among vials containing lyophilized formulations prepared using 100 mM phosphate buffer solution, there appeared some vials containing lyophilized formulations
characterized in that the lyophilized cake after forced deteriorative storage (80°C, one week) has a property of shrinking or floating to upper side of vial. That is, lyophilized formulations prepared using phosphate buffer solution of high concentration showed morphological changes under the condition of forced deteriorative storage. In this context, "shrink" refers to a state of a lyophilized cake which is characterized by that lyophilized cake is shrunk as a result of, for example, partial melting of frozen water during lyophilization.

From the results above, it was proved that a lower phosphate concentration is preferred so as to maintain the stability of a lyophilized formulation. The above-mentioned results indicate that a 10 mM - 20 mM phosphate buffer solution is preferred.

### Example 5 (Formulation of 240 g scale)

A solution of 1 mg/mL tocopherol (E mix 80 or E mix 70L)/ 10 % ethanol/90 % heptane was prepared, and a portion (40 mL) thereof was mixed thoroughly with squalane (16 g) to give "mixture-B".

As bacterial cell wall skeleton, BCG-CWS (163 mg) was added to a mixed solution of mixture-B (4.2 g) and 10% ethanol/90% heptane (20 mL), and allowed to disperse by shaking or ultrasonication at room temperature. The dispersed mixture was then heated at 70 °C under nitrogen flow or atmospheric air to evaporate ethanol/heptane. After adding aqueous 0.02 %w/w Polysorbate 80/5.7 % mannitol solution (212 g), the mixture was roughly emulsified using a homomixer, which was followed by addition of aqueous 10 %w/w Polysorbate 80 solution (1.74 g) and then complete emulsification. The final concentration of Polysorbate 80 was adjusted to 1 %w/w by adding and mixing a solution of 10 w/w% Polysorbate 80 (21.8 g) to give an oil-in-water emulsion (final formulation) (240 g).

The resulting oil-in-water emulsion formulation was dispensed into 2 mL aliquots in vials, and lyophilized to provide the lyophilized formulation of the present invention. Lyophilization was carried out using a lyophilizer (DFM-05A-S ULVAC).

### Example 6 (Reference formulation of 240 g scale)

A lyophilized formulation was prepared in a similar manner to Example 5 without adding tocopherol.

### INDUSTRIAL APPLICABILITY

The lyophilized formulation comprising as an active ingredient bacteria-CWS of the present invention is useful as a therapeutic agent in cancer immunotherapy.

## Claims

1. A lyophilized formulation of an oil-in-water emulsion comprising bacterial cell wall skeleton, an oil and a surfactant, **characterized in that** it further comprises an antioxidant.

2. The lyophilized formulation according to claim 1, wherein the surfactant is polyoxyethylene sorbitan fatty acid ester.

3. The lyophilized formulation according to claim 1 or 2, wherein the antioxidant is tocopherol.

4. The lyophilized formulation according to any one of claims 1 to 3, **characterized in that** it further contains a buffer agent so that the pH of an oil-in-water emulsion comprising bacterial cell wall skeleton at a concentration of 0.5 - 1 mg/ml obtainable by rehydrating said lyophilized formulation in water is adjusted to pH 5.5-8.5.

5. The lyophilized formulation according to claim 4, wherein the buffer agent is a phosphate.

6. The lyophilized formulation according to claim 5, **characterized in that** it is adjusted so that the concentration of the buffer agent in an oil-in-water emulsion comprising bacterial cell wall skeleton at a concentration of 0.5 - 1 mg/ml obtainable by rehydrating said lyophilized formulation in water is about 10 - 20 mM.

7. The lyophilized formulation according to any one of claims 1 to 6, **characterized in that** it further comprises mannitol as an excipient.

8. The lyophilized formulation according to any one of claims 1 to 7, wherein the bacterial cell wall skeleton are *Mycobacterium bovis Bacille Calmette-Guerin* cell wall skeleton.

9. The lyophilized formulation according to any one of claims 1 to 8, which has characteristics (1) and (2) below:
(1) the amount of free mycolic acid after 30-day storage at 80°C is about 2 % or less, and
(2) the pH of an oil-in-water emulsion comprising bacterial cell wall skeleton at a concentration of 0.5 - 1 mg/ml obtainable by rehydrating said lyophilized formulation in water after 30-day storage at 80°C is substantially equal to the pH before storage.

10. An oil-in-water emulsion obtainable by adding an aqueous solvent to a lyophilized formulation described in any one of claims 1 to 9.

11. An oil-in-water emulsion comprising bacterial cell wall skeleton, an oil and a surfactant, **characterized in that** it further comprises an antioxidant.

12. The oil-in-water emulsion according to claim 11, wherein the surfactant is polyoxyethylene sorbitan fatty acid ester.

13. The oil-in-water emulsion according to claim 11 or 12, wherein the antioxidant is tocopherol;

14. The oil-in-water emulsion according to any one of claims 11 to 13, **characterized in that** it further contains a buffer agent and is adjusted to pH 5.5 - 8.5.

15. The oil-in-water emulsion according to claim 14, wherein the buffer agent is a phosphate.

16. The oil-in-water emulsion according to claim 14 or 15, **characterized in that** it is adjusted so that the concentration of the buffer agent is about 10 - 20 mM.

17. The oil-in-water emulsion according to any one of claims 11 to 16, **characterized in that** it further comprises mannitol as an excipient.

18. The oil-in-water emulsion according to any one of claims 11 to 17, wherein the bacterial cell wall skeleton are *Mycobacterium bovis Bacille Calmette-Guerin* cell wall skeleton.

19. A lyophilized formulation obtainable by lyophilizing an oil-in-water emulsion described in any one of claims 11 to 18.

20. A method of stabilizing the above-mentioned lyophilized formulation comprising bacterial cell wall skeleton, which comprises adding a stabilizing agent containing an antioxidant to the lyophilized formulation and thereby conferring a long-term storage stability on the above-mentioned bacterial cell wall skeleton in the lyophilized formulation.

21. The method according to claim 20, which further comprises adding a buffer agent so as to adjust the pH of an oil-in-water emulsion comprising bacterial cell wall skeleton at a concentration of 0.5 - 1 mg/ml obtainable by rehydrating said lyophilized formulation to pH 5.5 - 8.5.

22. A method of inhibiting degradation of a bacterial cell wall skeleton in a lyophilized formulation comprising bacterial cell wall skeleton, an oil and a surfactant, **characterized in that** an antioxidant is added to the lyophilized formulation.

23. The method according to any one of claims 20 to 22, wherein the bacterial cell wall skeleton is BCG-CWS, the oil is squalane, the surfactant is Polysorbate 80, and the antioxidant is tocopherol.

24. A stabilization accelerator for an oil-in-water emulsion comprising bacterial cell wall skeleton, squalane and a surfactant or a lyophilized formulation of the emulsion, **characterized in that** it comprises tocopherol as an active ingredient.

25. The stabilization accelerator according to claim 24, **characterized in that** it further comprises a buffer agent so that the pH of an oil-in-water emulsion before lyophilization which contains bacterial cell wall skeleton at a concentration of 0.5 - 1 mg/ml or the pH of an oil-in-water emulsion which is obtainable by rehydrating the lyophilized formulation and contains bacterial cell wall skeleton at a concentration of 0.5 - 1 mg/ml is adjusted to pH 5.5 - 8.5.

26. The stabilization accelerator according to claim 25, **characterized in that** the buffer agent is a phosphate.
